# EUROPEAN PATENT APPLICATION

(11) **EP 4 613 758 A1**
(43) Date of publication of application: **10.09.2025**
(21) Application number: 23899989.0
(22) Date of filing: 06.12.2023
(51) Int. Cl.: C07K 7/06, C07K 1/04, C07K 1/06, A61K 51/08, A61K 103/00, A61P 35/00

(54) **COMPOUNDS TARGETING SSTR2, PREPARATION METHOD THEREFOR, AND USE THEREOF**

(30) Priority: 09.12.2022 CN 202211576787
(71) Applicant: Yantai Lannacheng Biotechnology Co., Ltd., Yantai Shandong 264199 (CN)
(72) Inventor: CHEN, Xiaoyuan, Yantai, Shandong 264199 (CN); WU, Xiaoming, Yantai, Shandong 264199 (CN); XU, Pengfei, Yantai, Shandong 264199 (CN); HE, Tian, Yantai, Shandong 264199 (CN); YANG, Qingbao, Yantai, Shandong 264199 (CN)
(74) Representative: Wang, Bo
(86) International application number: PCT/CN2023/136628
(87) International publication number: WO 2024/120415

(57) **Abstract**

The present invention provides a compound targeting SSTR2, a preparation method therefor and a use thereof, and relates to the fields of nuclear medicine and molecular imaging. The compound targeting SSTR2 has a structure shown in a formula (I) below, and a compound targeting SSTR2 and capable of being labeled with a radionuclide has a structure shown in a formula (II) below. The present invention further provides a radionuclide labeled compound targeting SSTR2 that is obtained by labeling the compound shown in the formula (II) with a radionuclide. The present invention further provides methods for preparing the compounds shown in the formula (I) and the formula (II) and a use of the compounds in preparation of drugs for diagnosis and/or treatment of diseases characterized by over-expression of SSTR2.

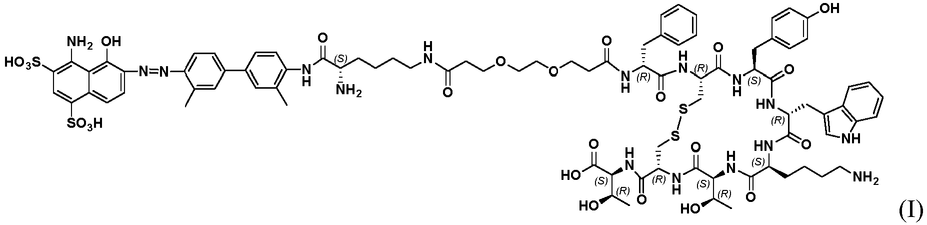

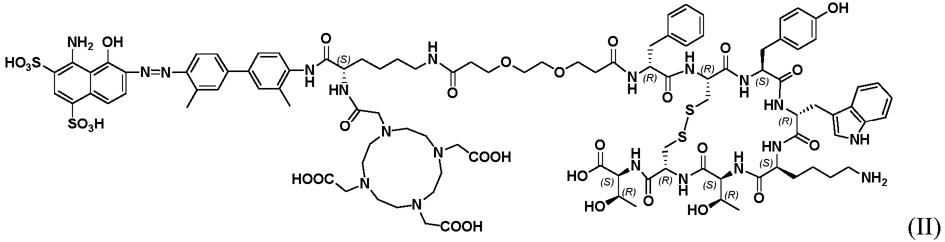

## Description

### TECHNICAL FIELD

The present invention relates to the fields of nuclear medicine and molecular imaging, and specifically relates to a compound targeting SSTR2, a preparation method therefor and a use thereof.

### BACKGROUND

Somatostatin receptor (SSTR) is a glycoprotein, which belongs to G protein-coupled receptors and has a total of 5 subtypes including SSTR1 to SSTR5. The SSTR affects an intracellular cAMP concentration by regulating the activity of an adenylate cyclase (AC), thereby transferring exogenous signals into cells. The SSTR participates in secretion regulation of a variety of hormones and is related to growth, proliferation and other processes of tumors. Researchers have found that the SSTR is highly expressed in a variety of tumor tissues, in which a somatostatin receptor 2 (SSTR2) is the most expressed. The SSTR2 can participate in many cell signal transduction pathways in organisms, such as downstream signals of GPCR and adenylate cyclase inhibition pathways. By changing a structure, the SSTR2 can activate downstream signal molecules, thereby regulating many physiological and pathological reaction processes in the organisms. The SSTR2 is frequently and highly expressed in a variety of tumor cells, especially in neuroendocrine tumors. In view of widespread expression and an important effect in tumors, the SSTR2 has become an important target for tumor imaging and therapy.

In recent years, peptide receptor radionuclide therapy (PRRT) has attracted wide attention. Radionuclide labeled peptide ligands specifically bind to receptors highly expressed in tumor cells to make chelated radionuclides transported to insides of the cells or adsorbed to surfaces of the cells, causing damage to the tumor cells through α rays or β rays released by nuclide decay. Lutathera (¹⁷⁷Lu-DOTA-TATE), PRRT targeting the somatostatin receptor 2 first proved by the FDA in 2018, has been used for treatment of inoperable or metastatic neuroendocrine tumors in the United States and several countries in Europe. However, due to rapid clearance of the Lutathera in the blood (mainly through the kidney), a dose of the Lutathera entering tumor tissues is greatly reduced, and the toxicity of kidney is also increased.

Previous studies have disclosed a long-acting somatostatin analog ¹⁷⁷Lu-EB-TATE, which can reversibly bind to albumin in vivo by introducing an azo dye (truncated EB) having high affinity to the albumin into a side chain of the Lutathera to prolong the half-life in the blood to 9.47 hours, about 4 times higher than that of the Lutathera. The ¹⁷⁷Lu-EB-TATE adopts a thiosuccinimide bond formed by maleimide-thiol as a coupling means, and the coupling has the advantages of high selectivity, fast reaction kinetics and mild reaction conditions, etc. However, the thiosuccinimide bond is easily eliminated through a retro-Michael reaction or a thiol exchange reaction under physiological conditions or in the presence of free thiol, resulting in poor in vivo stability. It is known in the art that because the succinimide bond is broken, a target portion is separated from a nuclide chelating portion, and a targeted delivery capability of a nuclide cannot be achieved. Especially for long-circulating PRRT drugs, due to a longer blood circulation time, higher in vivo stability is required to ensure full binding to highly expressed receptors at tumor sites. Therefore, it is necessary to improve a coupling bond of EB-TATE to improve the in vivo stability, increase a tumor uptake dose and meet demands of nuclide therapy and imaging.

### SUMMARY

In view of the above background, in order to solve the problem of poor in vivo stability of peptide receptor radionuclide therapeutic drugs, a primary purpose of the present invention is to develop a novel radionuclide labeled SSTR2-targeting compound, which can improve in vivo stability and increase a tumor uptake dose by coupling an EB portion and a TATE portion through an amide bond.

Another purpose of the present invention is to provide a method for preparing the novel compound, so as to complete preparation in a simple and efficient synthesis route.

Further another purpose of the present invention is to provide a use of the compound in diagnosis or treatment of diseases characterized by over-expression of SSTR2.

The above purposes of the present invention are achieved through the following technical schemes.

In a first aspect, the present invention provides a compound capable of targeting SSTR2 and having a structure shown in a formula (I), where the compound structurally includes an SSTR2 binding ligand and truncated Evans blue (namely truncated EB) simultaneously, and the SSTR2 binding ligand and the truncated Evans blue are linked by a flexible connecting arm containing an amide bond:

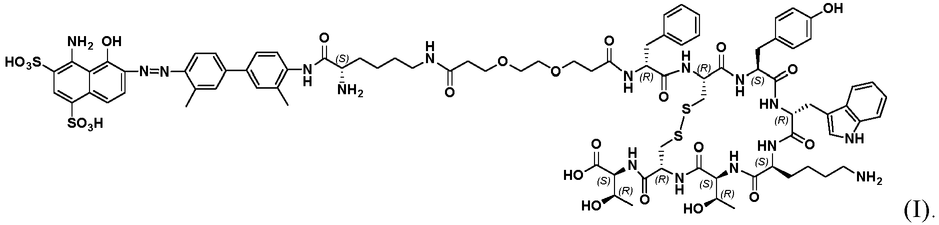

In a second aspect, the present invention provides a compound targeting SSTR2 and capable of being labeled with a radionuclide, where the compound structurally includes an SSTR2 binding ligand, truncated Evans blue, a flexible connecting arm and a nuclide chelating structure simultaneously, and the compound has a structure shown in a formula (II) below:

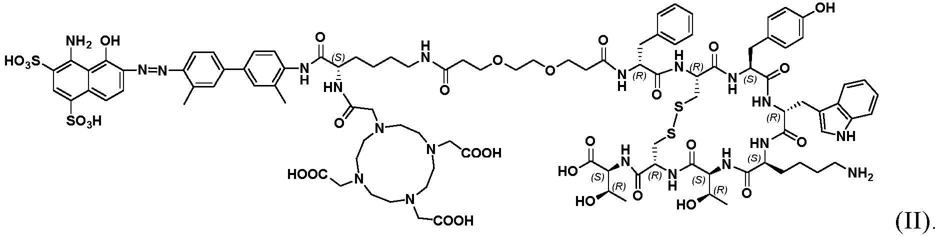

In a third aspect, the present invention provides a radionuclide labeled compound targeting SSTR2, which is obtained by labeling the compound having the structure shown in the formula (II) according to the second aspect of the present invention with a radionuclide.

In the schemes of the present invention, the radionuclide may be selected from isotopes emitting α rays, isotopes emitting β rays, isotopes emitting γ rays, isotopes emitting Auger electrons, or isotopes emitting X rays, etc., such as any one of ⁵¹Cr, ⁶⁷Ga, ⁶⁸Ga, ¹¹¹In, ^{99m}Tc, ¹⁸⁶Re, ¹⁸⁸Re, ¹³⁹La, ¹⁴⁰La, ¹⁷⁵Yb, ¹⁵³Sm, ¹⁶⁶Ho, ⁸⁶Y, ⁹⁰Y, ¹⁴⁹Pm, ¹⁶⁵Dy, ¹⁶⁹Er, ¹⁷⁷Lu, ⁴⁷Sc, ¹⁴²Pr, ¹⁵⁹Gd, ²¹²Bi, ²¹³Bi, ⁷²As, ⁷²Se, ⁹⁷Ru, ¹⁰⁹Pd, ¹⁰⁵Rh, ^{101m}Rh, ¹¹⁹Sb, ¹²⁸Ba, ¹²³I, ¹²⁴I, ¹³¹I, ¹⁹⁷Hg, ²¹¹At, ¹⁵¹Eu, ¹⁵³Eu, ¹⁶⁹Eu, ²⁰¹Tl, ²⁰³Pb, ²¹²Pb, ⁶⁴Cu, ⁶⁷Cu, ¹⁹⁸Au, ²²⁵Ac, ²²⁷Th, ⁸⁹Zr, or ¹⁹⁹Ag; and the radionuclide is more preferably ¹⁷⁷Lu, ²²⁵Ac, ⁶⁸Ga, ⁶⁴Cu, ⁸⁹Zr, ⁹⁰Y, ^{99m}Tc, ¹⁸⁸Re, or ¹¹¹In.

In a fourth aspect, the present invention provides a method for preparing the radionuclide labeled compound targeting SSTR2 according to the third aspect, where the preparation method provided by the present invention includes:
(1) swelling 10.05 g of a 2-CTC resin as a starting material, and adding 10.78 g of Fmoc-O-tert-butyl-L-threonine and 11.17 g of N,N-diisopropylethylamine to undergo a reaction; performing rinsing with dichloromethane first, then using dichloromethane, methanol and N,N-diisopropylethylamine to undergo a reaction for end capping, sequentially performing washing with dichloromethane, methanol and N,N-dimethylformamide, and then adding 100 mL of an N,N-dimethylformamide solution containing 20% of piperidine to remove an Fmoc group; after performing washing, adding 6.84 g of benzotriazole-N,N,N',N'-tetramethyluronium hexafluorophosphate, 0.23 g of 1-hydroxybenzotriazole (HOBT), 10.55 g of Fmoc-S-triphenylmethyl-L-cysteine and 2.37 g of N,N-diisopropylethylamine, and performing suction filtration after a reaction is completed; sequentially performing washing with dichloromethane, methanol and N,N-dimethylformamide, and then adding 100 mL of an N,N-dimethylformamide solution containing 20% of piperidine to remove an Fmoc group; after performing washing, adding 6.84 g of benzotriazole-N,N,N',N'-tetramethyluronium hexafluorophosphate, 0.18 g of 1-hydroxybenzotriazole, 7.17 g of Fmoc-O-tert-butyl-L-threonine and 2.37 g of N,N-diisopropylethylamine, and performing suction filtration after a reaction is completed; sequentially performing washing with dichloromethane, methanol and N,N-dimethylformamide, and then adding 100 mL of an N,N-dimethylformamide solution containing 20% of piperidine to remove an Fmoc group; after performing washing, adding 6.84 g of benzotriazole-N,N,N',N'-tetramethyluronium hexafluorophosphate, 0.18 g of 1-hydroxybenzotriazole, 8.45 g of Nε-(tert-butoxycarbonyl)-Nα-[(9H-fluoren-9-ylmethoxy)carbonyl]-L-lysine and 2.37 g of N,N-diisopropylethylamine, and performing suction filtration after a reaction is completed; sequentially performing washing with dichloromethane, methanol and N,N-dimethylformamide, and then adding 100 mL of an N,N-dimethylformamide solution containing 20% of piperidine to remove an Fmoc group; after performing washing, adding 6.84 g of benzotriazole-N,N,N',N'-tetramethyluronium hexafluorophosphate, 0.12 g of 1-hydroxybenzotriazole, 9.48 g of 1-[(1,1-dimethylethoxy)carbonyl]-N-[(9H-fluoren-9-methoxy)carbonyl]-D-tryptophan and 2.37 g of N,N-diisopropylethylamine, and performing suction filtration after a reaction is completed; sequentially performing washing with dichloromethane, methanol and N,N-dimethylformamide, and then adding 100 mL of an N,N-dimethylformamide solution containing 20% of piperidine to remove an Fmoc group; after performing washing, adding 6.84 g of benzotriazole-N,N,N',N'-tetramethyluronium hexafluorophosphate, 0.18 g of 1-hydroxybenzotriazole, 8.28 g of Fmoc-O-tert-butyl-L-tyrosine and 2.37 g of N,N-diisopropylethylamine, and performing suction filtration after a reaction is completed; sequentially performing washing with dichloromethane, methanol and N,N-dimethylformamide, and then adding 100 mL of an N,N-dimethylformamide solution containing 20% of piperidine to remove an Fmoc group; after performing washing, adding 6.84 g of benzotriazole-N,N,N',N'-tetramethyluronium hexafluorophosphate, 0.23 g of 1-hydroxybenzotriazole, 10.55 g of Fmoc-S-triphenylmethyl-L-cysteine and 2.37 g of N,N-diisopropylethylamine, and performing suction filtration after a reaction is completed; sequentially performing washing with dichloromethane, methanol and N,N-dimethylformamide, and then adding 100 mL of an N,N-dimethylformamide solution containing 20% of piperidine to remove an Fmoc group; after performing washing, adding 6.84 g of benzotriazole-N,N,N',N'-tetramethyluronium hexafluorophosphate, 0.14 g of 1-hydroxybenzotriazole, 6.97 g of Fmoc-D-phenylalanine and 2.37 g of N,N-diisopropylethylamine, and performing suction filtration after a reaction is completed; sequentially performing washing with dichloromethane, methanol and N,N-dimethylformamide, adding 200 mL of N,N-dimethylformamide, then adding a mixture of 8.44 g of iodine, 10 mL of methanol and 10 mL of N,N-dimethylformamide to remove -TRT protection and realize cyclization, and adding 100 mL of an N,N-dimethylformamide solution containing 20% of piperidine to remove Fmoc protection; after performing washing, adding 60 mL of N,N-dimethylformamide, 3.42 g of 2-(7-azobenzotriazole)-N,N,N',N'-tetramethyluronium hexafluorophosphate, 1.16 g of N,N-diisopropylethylamine and 7.43 g of (S)-5-((4'-amino-3,3'-dimethyl-[1,1'-diphenyl]-4-yl)carbonyl)-1-(9H-fluoren-9-yl)-3,11-diox o-2,14,17-trioxo-4,10-diazaicosane-20-acid, and performing suction filtration after a reaction is completed; sequentially performing washing with dichloromethane, methanol and N,N-dimethylformamide, and then adding 100 mL of an N,N-dimethylformamide solution containing 20% of piperidine to remove an Fmoc group; after performing washing, adding 60 mL of an N,N-dimethylformamide solution and 8.44 g of tri-tert-butyl 2,2',2"-(10-(2-((2,5-dioxopyrrolidin-1-yl)oxy)-2-oxoethyl)-1,4,7,10-tetraazacyclododecane-1 ,4,7-triyl)triacetate, performing suction filtration after a reaction is completed, and then cutting a resin with 200 mL of a dichloromethane solution containing 20% of hexafluoroisopropanol; after performing spin-drying, adding acetonitrile, water and 4.5 mL of a 2 M HCl solution, adding 25 mL of N,N-dimethylformamide for dissolution, performing cooling, adding an aqueous solution of 0.75 g of sodium nitrite, adding 3.38 g of a 1-amino-8-naphthol-2,4-disulfonic acid monosodium salt and an aqueous solution of 11.34 g of sodium bicarbonate at low temperature, performing spin-drying after a reaction is completed, and performing purification by preparative separation to obtain an intermediate; and then adding the obtained intermediate to a 95% trifluoroacetic acid aqueous solution for deprotection, and performing purification by preparative liquid phase separation to obtain a target product, namely the compound having the structure shown in the formula (II) according to the second aspect of the present invention; and
(2) enabling the compound capable of being labeled with a radionuclide obtained in step (1) to undergo a reaction with a compound containing a radionuclide by a wet labeling method or a freeze-drying labeling method to prepare and obtain the radionuclide labeled compound targeting SSTR2 according to the third aspect of the present invention.

In a fifth aspect, the present invention provides a pharmaceutical composition, where the pharmaceutical composition includes the compound targeting SSTR2 and capable of being labeled with a radionuclide according to the second aspect of the present invention or the radionuclide labeled compound targeting SSTR2 according to the third aspect of the present invention, or their pharmaceutically acceptable hydrates, solvates or salts.

In a sixth aspect, the present invention further provides a use of the compound targeting SSTR2 and capable of being labeled with a radionuclide according to the second aspect of the present invention, the radionuclide labeled compound targeting SSTR2 according to the third aspect of the present invention, or the pharmaceutical composition according to the fifth aspect in preparation of drugs for diagnosis or treatment of diseases characterized by over-expression of SSTR2 in animals or human individuals.

In the use of the present invention, the diseases characterized by over-expression of SSTR2 include, but are not limited to: neuroendocrine tumors, gastroenteric tumors, lung cancer, hepatocellular carcinoma, nasopharyngeal carcinoma, breast cancer, pancreatic cancer, small intestine cancer, colon cancer, rectal cancer, head and neck cancer, ovarian cancer, esophageal cancer, hypopharyngeal carcinoma, laryngeal carcinoma, myeloma cells, bladder cancer, cholangiocellular carcinoma, clear cell renal cell carcinoma, carcinogenic osteomalacia, sarcoma, cancer of unknown primary (CUP), thymic carcinoma, glioma, neuroglioma, astrocytoma, cervical cancer, or prostatic cancer.

Compared with the prior art, the compound targeting SSTR2 provided by the present invention has higher in vivo stability, combined with a long blood circulation time, can exhibit excellent metabolic kinetics, high tumor uptake and a long tumor retention time, and is expected to be applied in diagnosis or treatment of diseases characterized by over-expression of SSTR2.

In addition, the method for preparing the compound targeting SSTR2 provided by the present invention adopts a method combining solid phase synthesis and liquid phase synthesis, has a simple reaction route, simple operation, a short synthesis cycle and a high yield, and is suitable for industrial large-scale production.

### BRIEF DESCRIPTION OF DRAWINGS

FIG. 1 is a liquid phase diagram of TATE-EB-01 in Example 1 of the present invention.
FIG. 2 is a mass spectrogram of the TATE-EB-01 in Example 1 of the present invention.
FIG. 3 shows cellular uptake results of a ¹⁷⁷Lu labeled TATE-EB-01 complex of the present invention, where A shows cellular uptake experiment results of ¹⁷⁷Lu-TATE-EB-01 in AR42J tumor cells and cellular uptake results after blocking by TATE-EB-01; and B shows competitive binding experiment tests of TATE-EB-01 and DOTA-TATE in AR42J tumor cells.
FIG. 4 shows MicroPET imaging results of a ⁶⁸Ga labeled TATE-EB-01 complex at different time points after injection and a statistical chart of uptake results of tumors and vital organs in the present invention.
FIG. 5 shows MicroPET imaging results of ⁶⁸Ga labeled EB-TATE at different time points after injection and a statistical chart of uptake results of tumors and vital organs.
FIG. 6 shows MicroPET imaging results of ⁶⁸Ga-DOTA-TATE at different time points after injection and a statistical chart of uptake results of tumors and vital organs.
FIG. 7 shows MicroPET imaging results of unlabeled TATE-EB-01 and ⁶⁸Ga-DOTA-TATE that are injected into AR42J tumor mice simultaneously in an inhibition experiment.
FIG. 8 shows SPECT imaging results of a ¹⁷⁷Lu labeled TATE-EB-01 complex at different time points after injection and a statistical chart of uptake results of tumors and vital organs.
FIG. 9 shows SPECT imaging results of ¹⁷⁷Lu-EB-TATE at different time points after injection and a statistical chart of uptake results of tumors and vital organs.
FIG. 10 shows biological distribution of major organs and tumor tissues of AR42J tumor-bearing mice at 1, 4, 24, 48, 72, and 96 h after injection of ¹⁷⁷Lu-TATE-EB-01 of the present invention.
FIG. 11 shows biological distribution of major organs and tumor tissues of AR42J tumor-bearing mice at 1, 4, 24, 48, and 96 h after injection of ¹⁷⁷Lu-EB-TATE.
FIG. 12 shows biological distribution of major organs and tumor tissues of AR42J tumor-bearing mice at 1, 4, 24, and 48 h after injection of ¹⁷⁷Lu-DOTA-TATE.
FIG. 13 shows in vivo and in vitro stability of a ⁶⁸Ga labeled TATE-EB-01 complex of the present invention, where (A) shows the stability of ⁶⁸Ga-TATE-EB-01 after incubation in normal saline or a glutathione solution for 2 h and urine metabolism in mice for 2 h through HPLC analysis, respectively; and (B) shows the stability of ⁶⁸Ga-EB-TATE after incubation in normal saline or a glutathione solution for 2 h and urine metabolism in mice for 2 h through HPLC analysis, respectively.

### DETAILED DESCRIPTION OF EMBODIMENTS

The technical schemes of the present invention are further explained and described below in conjunction with specific embodiments and attached drawings.

Codes and structures of main compounds involved in the following examples are as follows:

| | |
|---|---|
| **TATE-EB-01** | 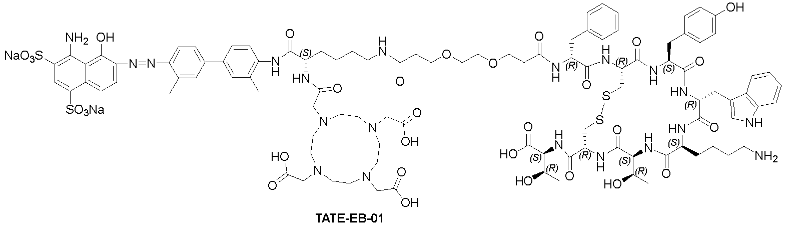 |
| **DOTA-TATE** | 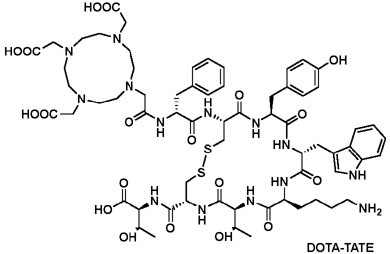 |
| **EB-TATE** | 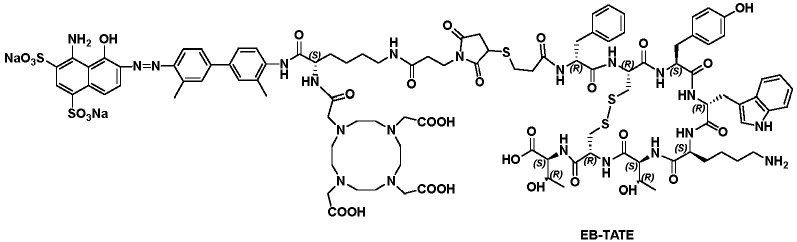 |

### Example 1 Preparation of a compound II

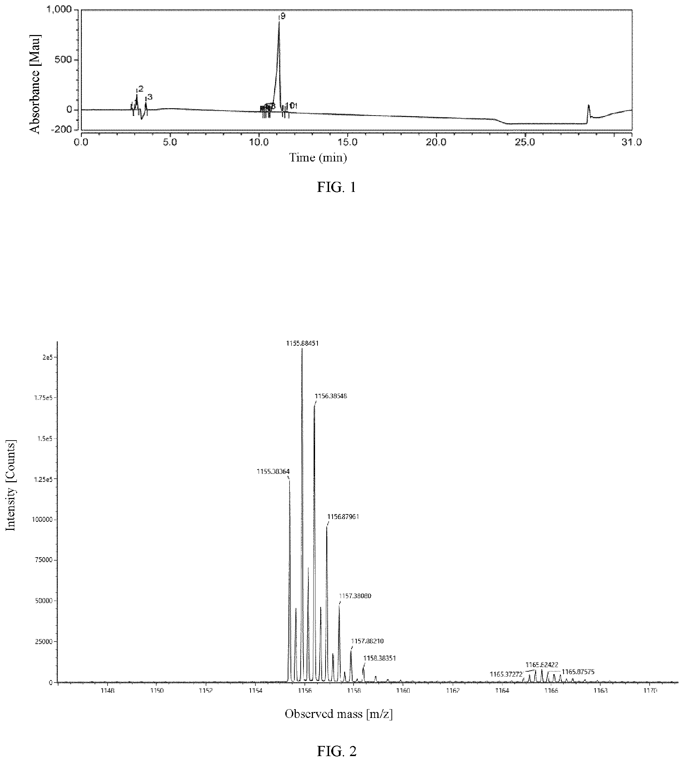

**Synthesis of an intermediate 21 by an Fmoc solid phase synthesis strategy:** 10.05 g of a 2-CTC resin as a starting material was swelled, and 10.78 g of Fmoc-O-tert-butyl-L-threonine (Fmoc-Thr(tBu)-OH) and 11.17 g of N,N-diisopropylethylamine were added to undergo a reaction to obtain an intermediate 1. Rinsing was performed with dichloromethane, then dichloromethane, methanol and N,N-diisopropylethylamine were used to undergo a reaction for end capping, washing was sequentially performed with dichloromethane, methanol and N,N-dimethylformamide, and then 100 mL of an N,N-dimethylformamide solution containing 20% of piperidine was added to remove an Fmoc group to obtain an intermediate 2. After washing was performed, 6.84 g of benzotriazole-N,N,N',N'-tetramethyluronium hexafluorophosphate, 0.23 g of 1-hydroxybenzotriazole (HOBT), 10.55 g of Fmoc-S-triphenylmethyl-L-cysteine and 2.37 g of N,N-diisopropylethylamine were added, and suction filtration was performed after a reaction was completed to obtain an intermediate 3. Washing was sequentially performed with dichloromethane, methanol and N,N-dimethylformamide, and then 100 mL of an N,N-dimethylformamide solution containing 20% of piperidine was added to remove an Fmoc group to obtain an intermediate 4. After washing was performed, 6.84 g of benzotriazole-N,N,N',N'-tetramethyluronium hexafluorophosphate, 0.18 g of 1-hydroxybenzotriazole, 7.17 g of Fmoc-O-tert-butyl-L-threonine and 2.37 g of N,N-diisopropylethylamine were added, and suction filtration was performed after a reaction was completed to obtain an intermediate 5. Washing was sequentially performed with dichloromethane, methanol and N,N-dimethylformamide, and then 100 mL of an N,N-dimethylformamide solution containing 20% of piperidine was added to remove an Fmoc group to obtain an intermediate 6. After washing was performed, 6.84 g of benzotriazole-N,N,N',N'-tetramethyluronium hexafluorophosphate, 0.18 g of 1-hydroxybenzotriazole, 8.45 g of Nε-(tert-butoxycarbonyl)-Nα-[(9H-fluoren-9-ylmethoxy)carbonyl]-L-lysine and 2.37 g of N,N-diisopropylethylamine were added, and suction filtration was performed after a reaction was completed to obtain an intermediate 7. Washing was sequentially performed with dichloromethane, methanol and N,N-dimethylformamide, and then 100 mL of an N,N-dimethylformamide solution containing 20% of piperidine was added to remove an Fmoc group to obtain an intermediate 8. After washing was performed, 6.84 g of benzotriazole-N,N,N',N'-tetramethyluronium hexafluorophosphate, 0.12 g of 1-hydroxybenzotriazole, 9.48 g of 1-[(1,1-dimethylethoxy)carbonyl]-N-[(9H-fluoren-9-methoxy)carbonyl]-D-tryptophan and 2.37 g of N,N-diisopropylethylamine were added, and suction filtration was performed after a reaction was completed to obtain an intermediate 9. Washing was sequentially performed with dichloromethane, methanol and N,N-dimethylformamide, and then 100 mL of an N,N-dimethylformamide solution containing 20% of piperidine was added to remove an Fmoc group to obtain an intermediate 10. After washing was performed, 6.84 g of benzotriazole-N,N,N',N'-tetramethyluronium hexafluorophosphate, 0.18 g of 1-hydroxybenzotriazole, 8.28 g of Fmoc-O-tert-butyl-L-tyrosine and 2.37 g of N,N-diisopropylethylamine were added, and suction filtration was performed after a reaction was completed to obtain an intermediate 11. Washing was sequentially performed with dichloromethane, methanol and N,N-dimethylformamide, and then 100 mL of an N,N-dimethylformamide solution containing 20% of piperidine was added to remove an Fmoc group to obtain an intermediate 12. After washing was performed, 6.84 g of benzotriazole-N,N,N',N'-tetramethyluronium hexafluorophosphate, 0.23 g of 1-hydroxybenzotriazole, 10.55 g of Fmoc-S-triphenylmethyl-L-cysteine and 2.37 g of N,N-diisopropylethylamine were added, and suction filtration was performed after a reaction was completed to obtain an intermediate 13. Washing was sequentially performed with dichloromethane, methanol and N,N-dimethylformamide, and then 100 mL of an N,N-dimethylformamide solution containing 20% of piperidine was added to remove an Fmoc group to obtain an intermediate 14. After washing was performed, 6.84 g of benzotriazole-N,N,N',N'-tetramethyluronium hexafluorophosphate, 0.14 g of 1-hydroxybenzotriazole, 6.97 g of Fmoc-D-phenylalanine and 2.37 g of N,N-diisopropylethylamine were added, and suction filtration was performed after a reaction was completed to obtain an intermediate 15. Washing was sequentially performed with dichloromethane, methanol and N,N-dimethylformamide, 200 mL of N,N-dimethylformamide was added, then a mixture of 8.44 g of iodine, 10 mL of methanol and 10 mL of N,N-dimethylformamide was added to remove -TRT protection and realize cyclization to obtain an intermediate 16, and 100 mL of an N,N-dimethylformamide solution containing 20% of piperidine was added to remove Fmoc protection to obtain an intermediate 17. After washing was performed, 60 mL of N,N-dimethylformamide, 3.42 g of 2-(7-azobenzotriazole)-N,N,N',N'-tetramethyluronium hexafluorophosphate, 1.16 g of N,N-diisopropylethylamine and 7.43 g of (S)-5-((4'-amino-3,3'-dimethyl-[1,1'-diphenyl]-4-yl)carbonyl)-1-(9H-fluoren-9-yl)-3,11-diox o-2,14,17-trioxo-4,10-diazaicosane-20-acid were added, and suction filtration was performed after a reaction was completed to obtain an intermediate 18. Washing was sequentially performed with dichloromethane, methanol and N,N-dimethylformamide, and then 100 mL of an N,N-dimethylformamide solution containing 20% of piperidine was added to remove an Fmoc group to obtain an intermediate 19. After washing was performed, 60 mL of an N,N-dimethylformamide solution and 8.44 g of tri-tert-butyl 2,2',2''-(10-(2-((2,5-dioxopyrrolidin-1-yl)oxy)-2-oxoethyl)-1,4,7,10-tetraazacyclododecane-1 ,4,7-triyl)triacetate were added, suction filtration was performed after a reaction was completed to obtain an intermediate 20, and then a resin was cut with 200 mL of a dichloromethane solution containing 20% of hexafluoroisopropanol to obtain an intermediate 20' (namely the intermediate 20 cut from the resin). After spin-drying was performed, acetonitrile, water and 4.5 mL of a 2 M HCl solution were added, 25 mL of N,N-dimethylformamide was added for dissolution, cooling was performed, an aqueous solution of 0.75 g of sodium nitrite was added, 3.38 g of a 1-amino-8-naphthol-2,4-disulfonic acid monosodium salt and an aqueous solution of 11.34 g of sodium bicarbonate were added at low temperature, spin-drying was performed after a reaction was completed, and purification was performed by preparative separation to obtain an intermediate 21. Then, the obtained intermediate 21 was added to 200 mL of a 95% trifluoroacetic acid aqueous solution for deprotection, and purification was performed by preparative liquid phase separation to obtain a target product TATE-EB-01 (namely the compound of the formula (II) of the present invention). FIG. 1 is a liquid phase diagram of the TATE-EB-01 in the present example. FIG. 2 is a mass spectrogram of the TATE-EB-01 in the present example. A theoretical molecular weight is [M+K-3H]²⁻/2=1155.89235, and a measured molecular weight is [M+K-3H]²⁻/2=1155.88451.

A synthetic route of the above steps is as follows:

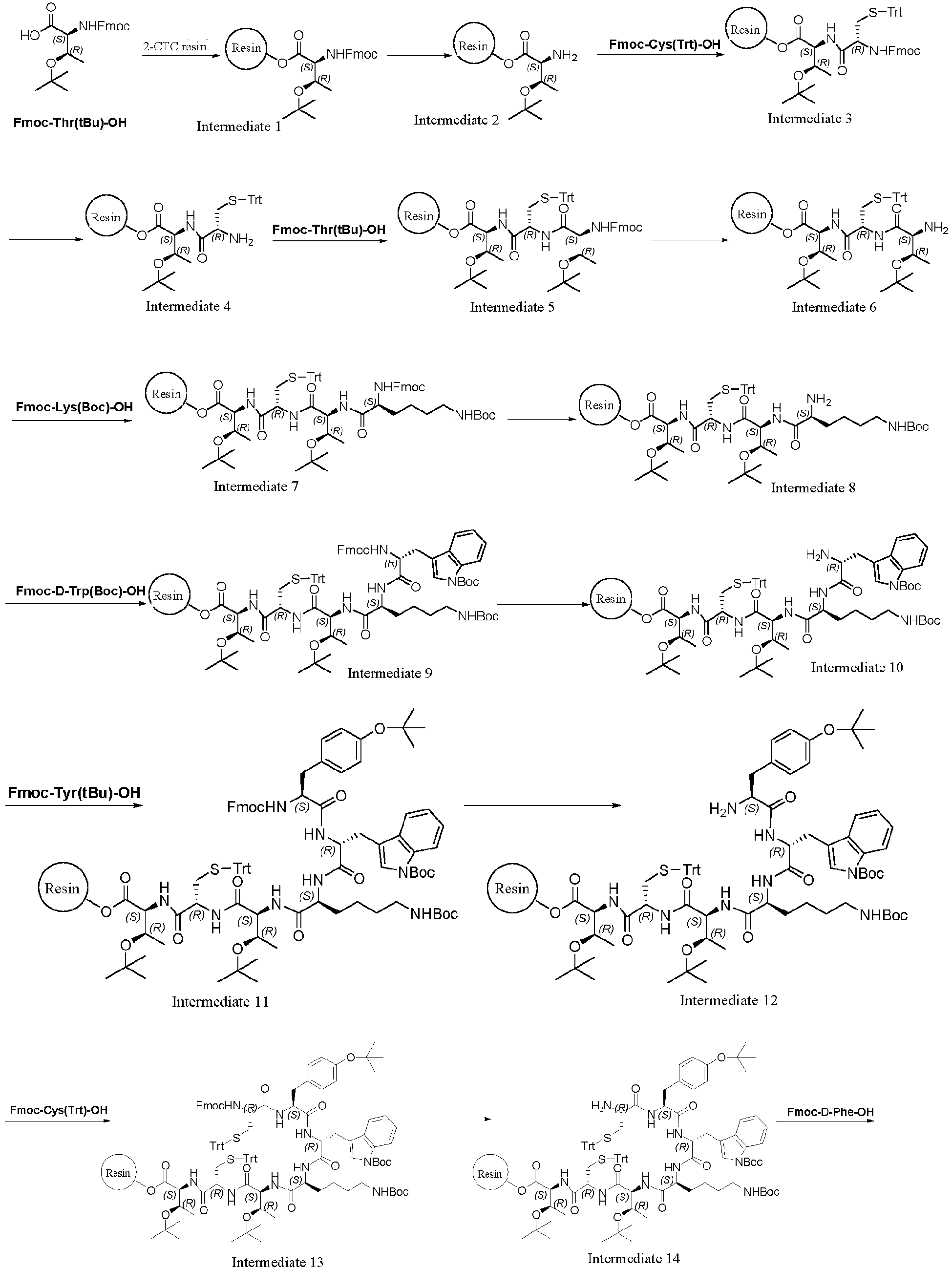

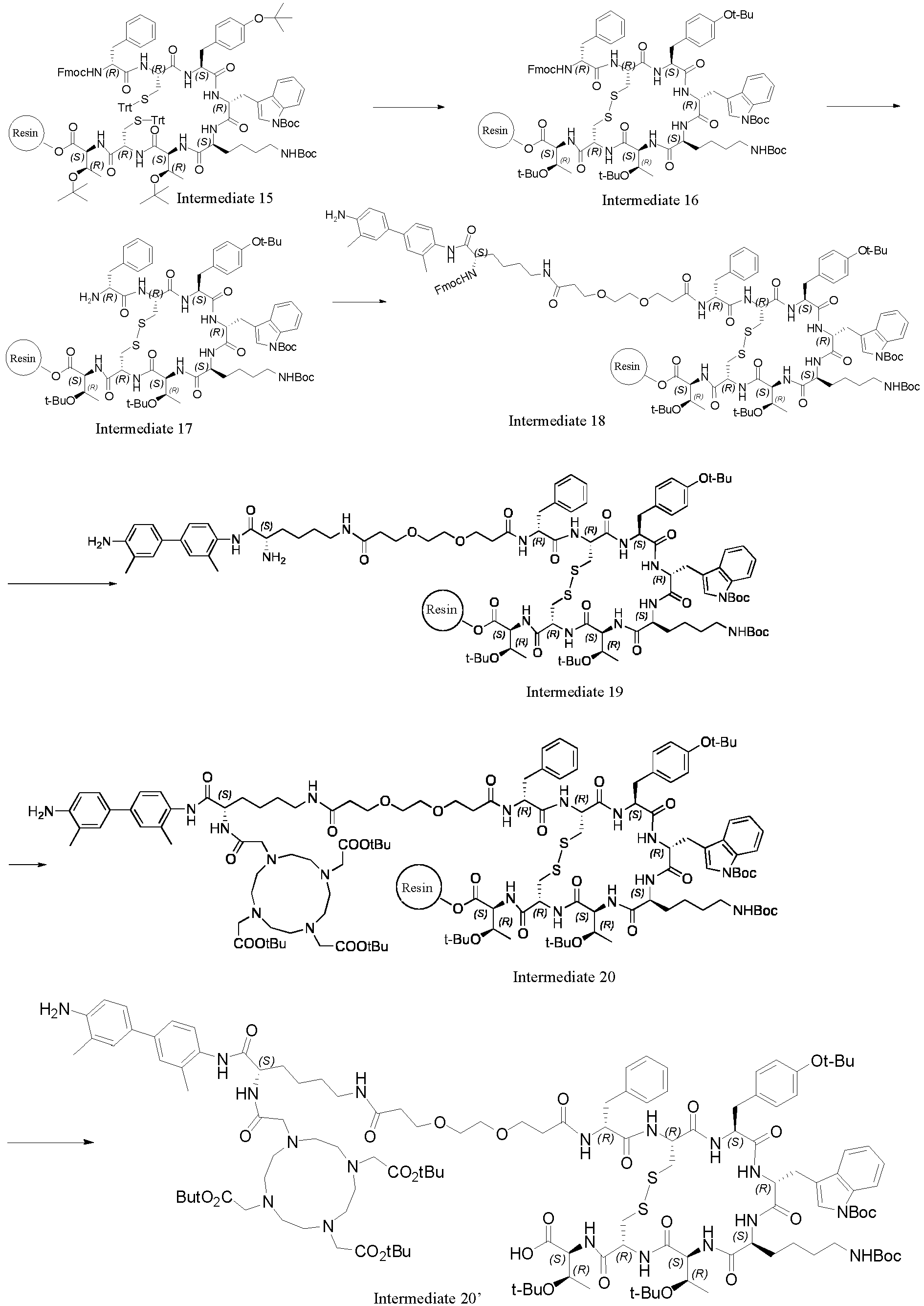

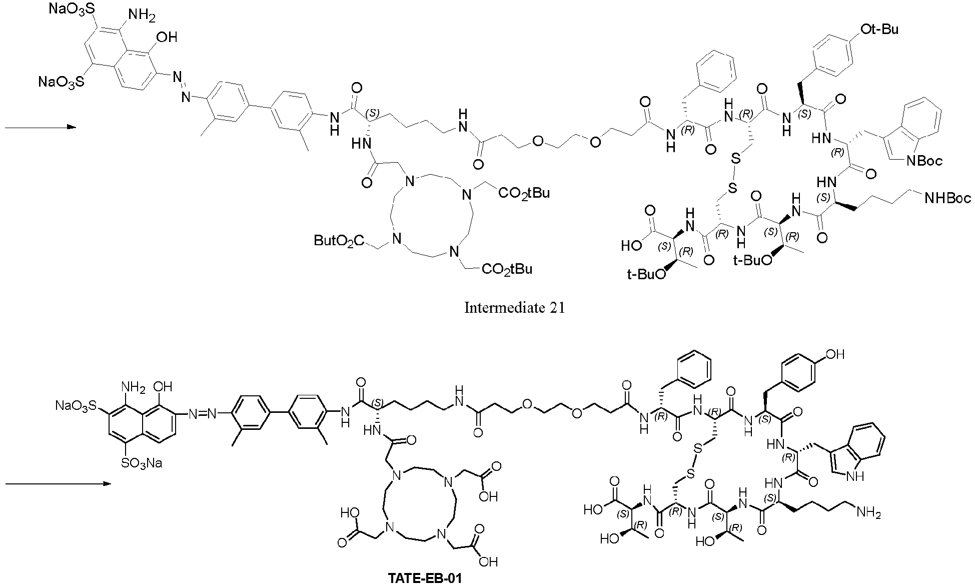

### Example 2 Preparation of a radioactive Ga-68 labeled TATE-EB-01 complex (⁶⁸Ga-TATE-EB-01)

**Wet method:** A hydrochloric acid solution of about 18.5-1,850 MBq of ⁶⁸GaCl₃ (rinsed with a germanium-gallium generator) was added into a centrifuge tube containing 0.5 mL of an acetic acid-acetate solution (1.0 g/L) of the compound TATE-EB-01 prepared in Example 1 to carry out a reaction at 95°C for 20 min. A small C₁₈ separation column was taken, slowly rinsed with 10 mL of anhydrous ethanol first, and then rinsed with 10 mL of water. After being diluted with 10 mL of water, a labeled solution was loaded to the separation column, treated with 10 mL of water to remove unlabeled ⁶⁸Ga ions first, and then rinsed with 0.3 mL of a 10 mM ethanol solution of HCl to obtain a ⁶⁸Ga labeled complex. The rinsed solution was diluted with normal saline and then subjected to sterile filtration to obtain an injection of a ⁶⁸Ga labeled TATE-EB-01 complex (recorded as ⁶⁸Ga-TATE-EB-01).

**Freeze-drying method:** A hydrochloric acid solution of about 18.5-1,850 Mbq of ⁶⁸GaCl₃ (rinsed with a germanium-gallium generator) was added into a freeze-dried medicine kit containing the compound TATE-EB-01 and mixed uniformly to carry out a reaction at 95°C for 20 min. A small C₁₈ separation column was taken, slowly rinsed with 10 mL of anhydrous ethanol first, and then rinsed with 10 mL of water. After being diluted with 10 mL of water, a labeled solution was loaded to the separation column, treated with 10 mL of water to remove unlabeled ⁶⁸Ga ions first, and then rinsed with 0.3 mL of a 10 mM ethanol solution of HCl to obtain a rinsed solution of a complex. The rinsed solution was diluted with normal saline and then subjected to sterile filtration to obtain an injection of a ⁶⁸Ga labeled TATE-EB-01 complex (recorded as ⁶⁸Ga-TATE-EB-01).

### Example 3 Preparation of a radioactive Lu-177 labeled TATE-EB-01 complex (¹⁷⁷Lu-TATE-EB-01)

Preparation of a buffer solution with a pH value of 5.5: 57.6 mg of acetic acid, 189 mg of gentisic acid and 525 mg of sodium acetate trihydrate were weighed and dissolved in 48 ml of pure water, and the pH value was adjusted to 5.5 with a sodium hydroxide solution. 200 µg of the compound TATE-EB-01 prepared in Example 1 was fully dissolved in 200 µL of the buffer solution (pH=5.5), and then 5 ml of the buffer solution (pH=5.5) and a hydrochloric acid solution of about 150 mCi of ¹⁷⁷LuCl₃ were added. A mixture was shaken uniformly and then heated to carry out a reaction at 80°C for 20 min. After the reaction was completed, cooling was performed to room temperature. A reaction solution was diluted with normal saline and then subjected to sterile filtration to obtain an injection of a ¹⁷⁷Lu labeled TATE-EB-01 complex at 10 mCi/mL (recorded as ¹⁷⁷Lu-TATE-EB-01).

### Experimental Example 1 Cellular uptake experiment

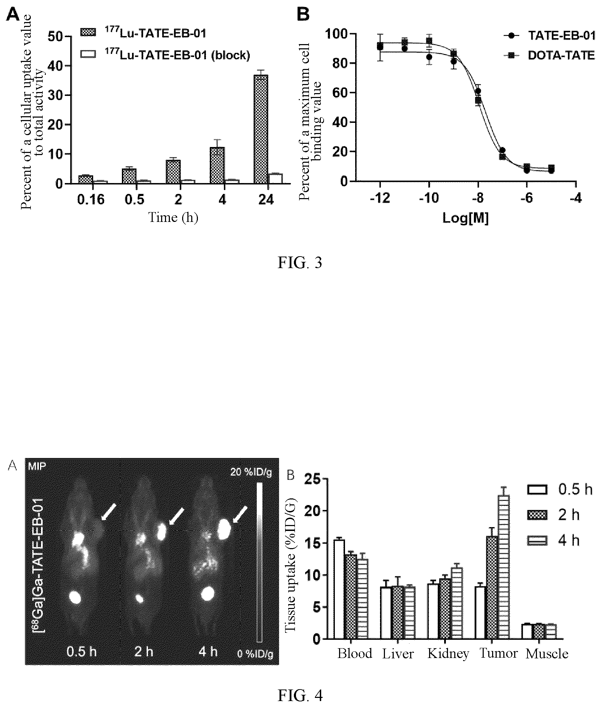

In a cellular uptake experiment, 2×10⁵AR42J tumor cells were inoculated into a 24-well plate overnight until the cells were adherent, and then the cells and 37 kBq of the ¹⁷⁷Lu-TATE-EB-01 prepared in Example 3 were incubated in 0.5 mL of a culture medium at 37°C for 0.16, 0.5, 2, 4, and 24 h. In a blocking experiment, unlabeled TATE-EB-01 (50 µg/mL) was added as an inhibitor to verify the targeting specificity. In a competitive binding experiment, AR42J tumor cells were incubated with different concentrations (10⁻⁵ to 10⁻¹² M) of unlabeled TATE-EB-01 (namely the compound of the formula II provided by the present invention) or DOTA-TATE and ¹⁷⁷Lu-DOTA-TATE (namely ¹⁷⁷Lu labeled DOTA-TATE) for 1 h. Then, a supernatant was removed, and the AR42J tumor cells were washed with cold PBS for two times and then lyzed with a 1 M NaOH solution. A cell lysate was collected and determined by a γ counter to obtain the radioactivity. The cellular uptake of ¹⁷⁷Lu-TATE-EB-01 is gradually increased over time, and the cellular uptake is the highest at 24 h, reaching 36.96±1.60%. When the unlabeled TATE-EB-01 is added as an inhibitor, the cellular uptake is decreased to 3.37±0.17%, indicating high binding specificity with SSTR2 (see A in FIG. 3). In addition, an IC₅₀ value of the TATE-EB-01 is 20.39 nM, and an IC₅₀ value of the DOTA-TATE is 11.48 nM, indicating that both the TATE-EB-01 and the DOTA-TATE have similar affinity with an SSTR2 receptor (see B in FIG. 3), which means that a modification strategy has little effect on binding affinity with the SSTR2 receptor.

### Experimental Example 2 MicroPET imaging of a ⁶⁸Ga labeled TATE-EB-01 complex in tumor-bearing mice

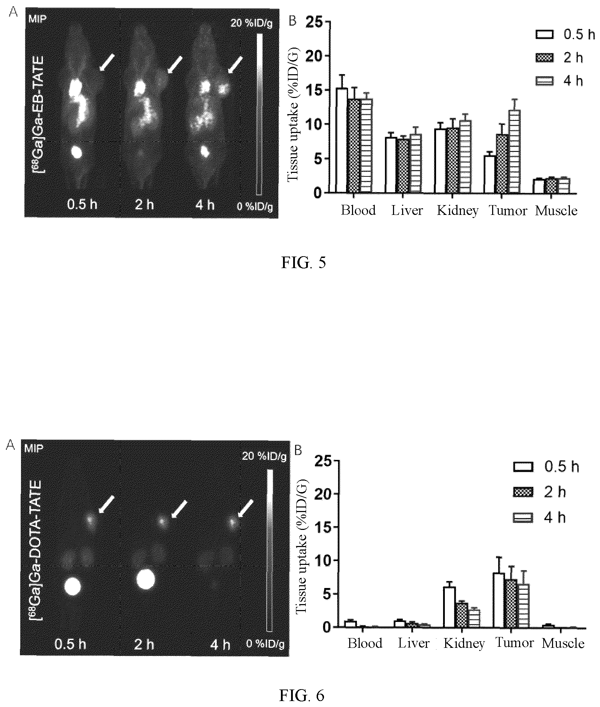

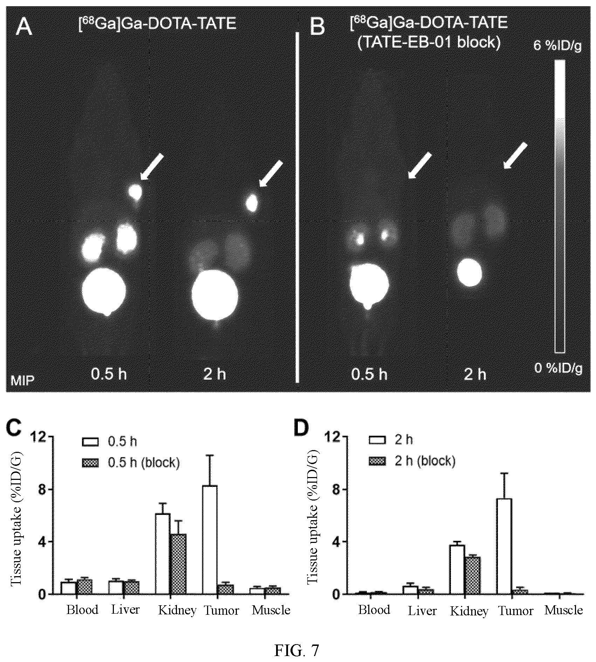

⁶⁸Ga-TATE-EB-01 was prepared according to the method in Example 2. In AR42J tumor-bearing mice, the mice that were randomly grouped were administered with 7.4 MBq of ⁶⁸Ga-TATE-EB-01, ⁶⁸Ga-EB-TATE (namely ⁶⁸GA labeled EB-TATE) and ⁶⁸Ga-DOTA-TATE (namely ⁶⁸GA labeled DOTA-TATE) through tail intravenous injection, respectively, and then, the tumor mice were anesthetized and placed on a PET/CT scanner for MicroPET imaging at 0-240 min after administration, respectively. A blocking experiment was carried out by simultaneously injecting unlabeled TATE-EB-01 and ⁶⁸Ga-DOTA-TATE into AR42J tumor mice and performing PET scanning. Results are shown in FIG. 4, FIG. 5, FIG. 6 and FIG. 7. Parts A in FIG. 4 to FIG. 6 show PET images of ⁶⁸Ga-TATE-EB-01, ⁶⁸Ga-EB-TATE and ⁶⁸Ga-DOTA-TATE after injection into AR42J tumor mice at time points of 0.5, 2, and 4 h, respectively. Parts B in FIG. 4 to FIG. 6 show quantitative analysis histograms of uptake values of major organs and tissues of mice after intravenous injection of ⁶⁸Ga-TATE-EB-01, ⁶⁸Ga-EB-TATE and ⁶⁸Ga-DOTA-TATE, respectively (quantitative analysis was performed with 3 mice in each group). As can be seen from Parts A in FIG. 4 to FIG. 6, tumors are clearly visible at the time points of imaging acquisition. As can be seen from Parts B in FIG. 4 to FIG. 6, the tumor uptake value of ⁶⁸Ga-TATE-EB-01 reaches 16.08±1.26%ID/g at 2 h after the injection and reaches 22.42±1.28%ID/g at 4 h after the injection, which is significantly higher than that of normal tissues (as shown in FIG. 4). In contrast, signals of the ⁶⁸Ga-EB-TATE in tumors are significantly lower than that of the ⁶⁸Ga-TATE-EB-01, and tumor uptake values at 2 and 4 h after the injection are 8.67±1.46%ID/g and 12.25±1.52%ID/g, respectively (as shown in FIG. 5). Tumor uptake amounts of the ⁶⁸Ga-DOTA-TATE at 2 and 4 h after the injection are 7.33±1.91%ID/g and 6.67±1.94%ID/g, respectively (as shown in FIG. 6). In order to evaluate the targeting specificity, the unlabeled TATE-EB-01 prepared in Example 1 and ⁶⁸Ga-DOTA-TATE were simultaneously injected into AR42J tumor mice to carry out an inhibition experiment. Results show that the unlabeled TATE-EB-01 can significantly inhibit tumor uptake (see Parts A and B in FIG. 7). Quantitative results of tumor uptake of corresponding drugs are shown in Parts C and D in FIG. 7. Compared with the PET imaging without inhibition, the tumor uptake value of a TT-EB-01 inhibition group in PET imaging is significantly lower.

### Experimental Example 3 SPECT imaging of a ¹⁷⁷Lu labeled TATE-EB-01 complex in tumor-bearing mice

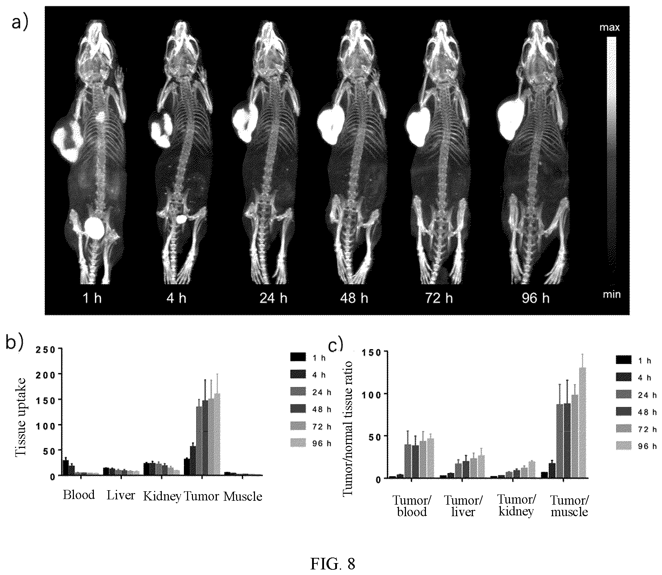

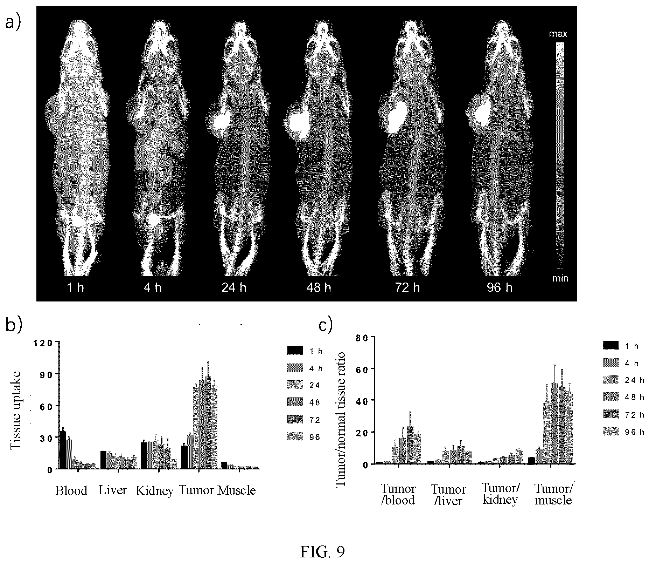

¹⁷⁷Lu-TATE-EB-01 was prepared according to the method in Example 3. In AR42J tumor-bearing mice, the mice that were randomly grouped were administered with 37 MBq of ¹⁷⁷Lu-TATE-EB-01 and ¹⁷⁷Lu-EB-TATE (namely ¹⁷⁷Lu labeled EB-TATE) through tail intravenous injection, respectively, and then subjected to SPECT imaging at 1-96 h after administration under anesthetization with isoflurane, respectively. Results are shown in FIG. 8 and FIG. 9. Parts a) in FIG. 8 and FIG. 9 show maximum density projection images of SPECT imaging of ¹⁷⁷Lu-TATE-EB-01 and ¹⁷⁷Lu-EB-TATE in AR42J tumor-bearing mice at different time points, respectively. As can be seen, tumors in the two groups are clearly visible at various time points of imaging acquisition, and the mice have a better SPECT imaging effect at various time points after injection of the ¹⁷⁷Lu-TATE-EB-01 by comparison. Parts b) in FIG. 8 and FIG. 9 show uptake of ¹⁷⁷Lu-TATE-EB-01 and ¹⁷⁷Lu-EB-TATE in various organs or tissues (blood, liver, kidney, tumor and muscle) of mice at different time points after intravenous injection, respectively, where three doses in each group correspond to, from left to right, 1 h, 4 h, 12 h, 24 h, 48 h, 72 h, and 96 h after the injection, respectively. As can be seen, the two drugs have similar distribution in the blood, liver, kidney and muscle at same time points, but the uptake of ¹⁷⁷Lu-TATE-EB-01 in the tumor is obviously higher than that of ¹⁷⁷Lu-EB-TATE at all time points. In addition, Parts c) in FIG. 8 and FIG. 9 show target/non-target ratios at different time points after intravenous injection of ¹⁷⁷Lu-TATE-EB-01 and ¹⁷⁷Lu-EB-TATE into mice. As can be seen, ratios of tumors to normal organs in the ¹⁷⁷Lu-TATE-EB-01 group at various time points are also significantly higher than that of the ¹⁷⁷Lu-EB-TATE. For example, at the test time point of 96 h, the tumor-muscle ratio of ¹⁷⁷Lu-TATE-EB-01 is higher than 120, while the tumor-muscle ratio of the ¹⁷⁷Lu-EB-TATE group is lower than 50. Therefore, the ¹⁷⁷Lu-TATE-EB-01 is significantly better than the ¹⁷⁷Lu-EB-TATE in both absolute tumor uptake and target/non-target ratio. In summary, the compound targeting SSTR2 of the present invention and a radionuclide labeled compound thereof exhibit excellent metabolokinetics, high tumor uptake and a long tumor retention time, and are expected to be applied in diagnosis or treatment of diseases characterized by over-expression of SSTR2.

### Experimental Example 4 Biological distribution

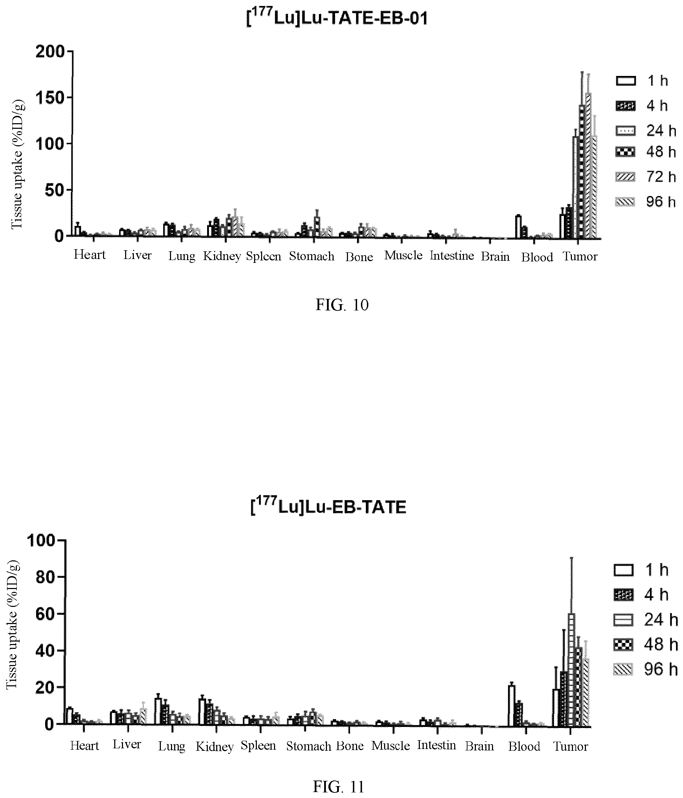

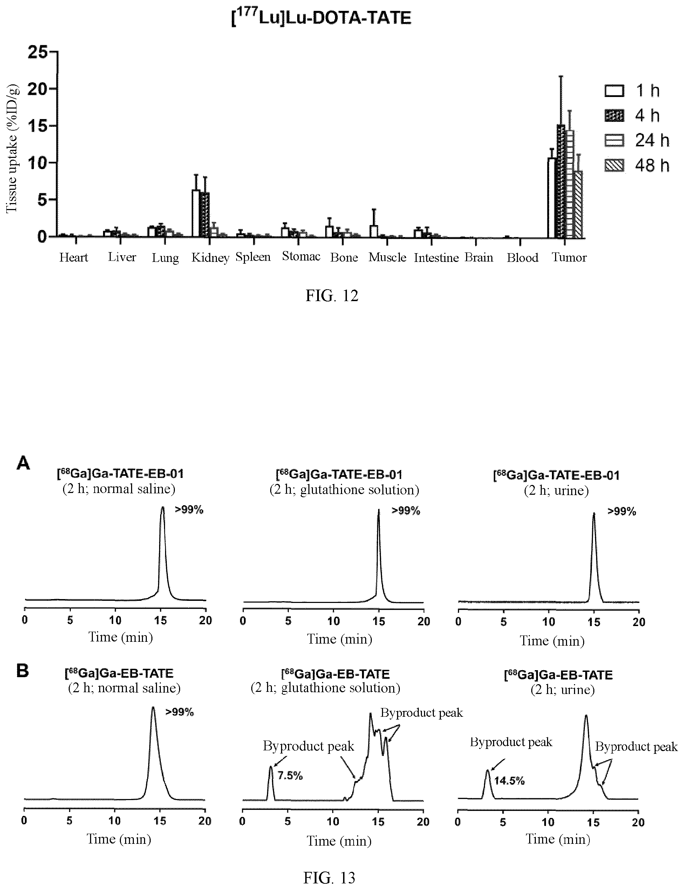

In a biological distribution study, AR42J tumor-bearing mice were administered with 1.48 Mbq of the ¹⁷⁷Lu-TATE-EB-01 prepared in Example 3, ¹⁷⁷Lu-EB-TATE or ¹⁷⁷Lu-DOTA-TATE (namely ¹⁷⁷Lu labeled DOTA-TATE) through intravenous injection, respectively, and dissected at different time points. Organs and tumor tissues of interest were collected and weighed. The radioactivity was detected by a γ counter, and biological distribution results were calculated as a percent of an injection dose per gram of tissue (%ID/g). Results are shown in FIG. 10. Tumor uptake of the ¹⁷⁷Lu-TATE-EB-01 prepared in Example 3 is gradually increased overtime and reaches a peak value at 72 h after the injection, with an uptake value of 158.32±20.04%ID/g, a high tumor uptake value of 112.44±20.57%ID/g is still maintained at 96 h after the injection, and the tumor uptake value is much higher than that of other organs, including a kidney uptake value (15.15±6.20%ID/g). In contrast, as shown in FIG. 11, it is observed that a tumor uptake value of existing ¹⁷⁷LuEB-TATE (37.28±9.50%ID/g at 96 h) is much lower than that of the ¹⁷⁷Lu-TATE-EB-01. As shown in FIG. 12, tumor intake of the ¹⁷⁷Lu-DOTA-TATE is lowest, which is only 9.19±2.16%ID/g at 48 h after the injection and is continuously decreased at 4 h after the injection. These results indicate that the ¹⁷⁷Lu-TATE-EB-01 of the present invention has highest tumor uptake and optimal in vivo distribution compared with the other two radionuclide labeled compounds.

### Experimental Example 5 Stability experiment

In order to explore causes for differences in distribution of radiolabeled TATE-EB-01 and EB-TATE in animals, ⁶⁸Ga-TATE-EB-01 and ⁶⁸Ga-EB-TATE were cultured in vitro in normal saline and a glutathone solution for 2 h, respectively, and then analyzed by radioactive high performance liquid chromatography. In addition, the ⁶⁸Ga-TATE-EB-01 and the ⁶⁸Ga-EB-TATE were injected into BALB/c nude mice, respectively, and urine samples were collected 2 h later and analyzed by radioactive high performance liquid chromatography. Results are shown in FIG. 13. The ⁶⁸Ga-TATE-EB-01 is almost maintained in an intact original drug form in the glutathione solution and urine 2 h later and has a radiochemical purity still greater than 99%. However, the ⁶⁸Ga-EB-TATE has poor stability in the glutathione solution and the urine at 2h, a plurality of byproduct peaks are generated, and the radiochemical purity of a main peak is obviously reduced, indicating that the ⁶⁸Ga-EB-TATE has poor stability in an in vitro reducing environment of glutathione and also has poor stability in animals, and the generation of byproducts at 2 h after the injection is also an indirect cause leading to poor pharmacokinetics of the probe in vivo.

Although the present invention has been described in detail through general description, specific embodiments and tests herein, it is obvious to persons skilled in the art that some modifications or improvements can be made on the basis of the present invention. Therefore, all the modifications or improvements that are made without deviating from the spirit of the present invention fall within the scope of protection required by the present invention.

## Claims

1. A compound targeting SSTR2, wherein the compound structurally comprises an SSTR2 binding ligand and truncated Evans blue simultaneously, and the SSTR2 binding ligand and the truncated Evans blue are linked by a flexible connecting arm containing an amide bond; and the compound has a structure shown in a formula (I):

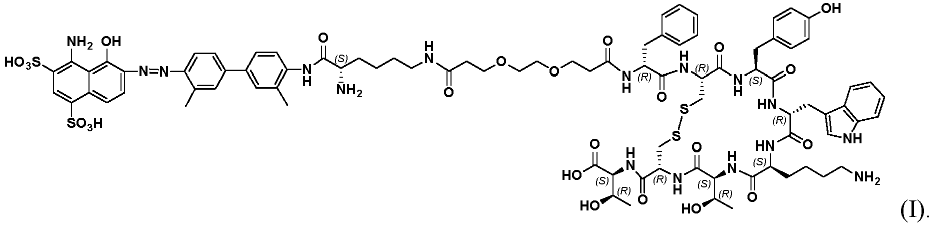

2. A compound targeting SSTR2 and capable of being labeled with a radionuclide, wherein the compound structurally comprises an SSTR2 binding ligand, truncated Evans blue, a flexible connecting arm and a nuclide chelating structure simultaneously, and the compound has a structure shown in a formula (II) below:

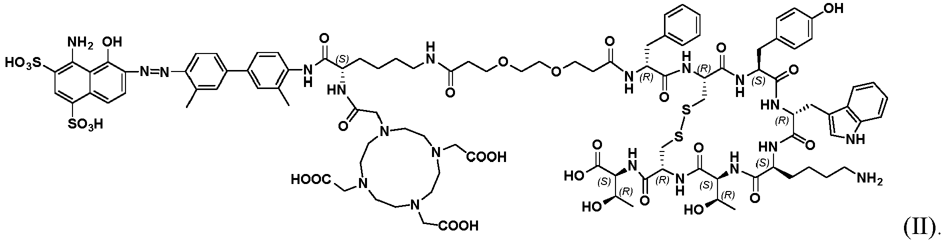

3. A method for preparing the compound targeting SSTR2 and capable of being labeled with a radionuclide according to claim 2, comprising synthesizing a precursor of a diazotization reaction by an Fmoc solid phase synthesis strategy and finally obtaining a target product by the diazotization reaction in one step, wherein the method specifically comprises the following steps:
swelling 10.05 g of a 2-CTC resin as a starting material, and adding 10.78 g of Fmoc-O-tert-butyl-L-threonine and 11.17 g of N,N-diisopropylethylamine to undergo a reaction; performing rinsing with dichloromethane first, then using dichloromethane, methanol and N,N-diisopropylethylamine to undergo a reaction for end capping, sequentially performing washing with dichloromethane, methanol and N,N-dimethylformamide, and then adding 100 mL of an N,N-dimethylformamide solution containing 20% of piperidine to remove an Fmoc group; after performing washing, adding 6.84 g of benzotriazole-N,N,N',N'-tetramethyluronium hexafluorophosphate, 0.23 g of 1-hydroxybenzotriazole, 10.55 g of Fmoc-S-triphenylmethyl-L-cysteine and 2.37 g of N,N-diisopropylethylamine, and performing suction filtration after a reaction is completed; sequentially performing washing with dichloromethane, methanol and N,N-dimethylformamide, and then adding 100 mL of an N,N-dimethylformamide solution containing 20% of piperidine to remove an Fmoc group; after performing washing, adding 6.84 g of benzotriazole-N,N,N',N'-tetramethyluronium hexafluorophosphate, 0.18 g of 1-hydroxybenzotriazole, 7.17 g of Fmoc-O-tert-butyl-L-threonine and 2.37 g of N,N-diisopropylethylamine, and performing suction filtration after a reaction is completed; sequentially performing washing with dichloromethane, methanol and N,N-dimethylformamide, and then adding 100 mL of an N,N-dimethylformamide solution containing 20% of piperidine to remove an Fmoc group; after performing washing, adding 6.84 g of benzotriazole-N,N,N',N'-tetramethyluronium hexafluorophosphate, 0.18 g of 1-hydroxybenzotriazole, 8.45 g of Nε-(tert-butoxycarbonyl)-Nα-[(9H-fluoren-9-ylmethoxy)carbonyl]-L-lysine and 2.37 g of N,N-diisopropylethylamine, and performing suction filtration after a reaction is completed; sequentially performing washing with dichloromethane, methanol and N,N-dimethylformamide, and then adding 100 mL of an N,N-dimethylformamide solution containing 20% of piperidine to remove an Fmoc group; after performing washing, adding 6.84 g of benzotriazole-N,N,N',N'-tetramethyluronium hexafluorophosphate, 0.12 g of 1-hydroxybenzotriazole, 9.48 g of 1-[(1,1-dimethylethoxy)carbonyl]-N-[(9H-fluoren-9-methoxy)carbonyl]-D-tryptophan and 2.37 g of N,N-diisopropylethylamine, and performing suction filtration after a reaction is completed; sequentially performing washing with dichloromethane, methanol and N,N-dimethylformamide, and then adding 100 mL of an N,N-dimethylformamide solution containing 20% of piperidine to remove an Fmoc group; after performing washing, adding 6.84 g of benzotriazole-N,N,N',N'-tetramethyluronium hexafluorophosphate, 0.18 g of 1-hydroxybenzotriazole, 8.28 g of Fmoc-O-tert-butyl-L-tyrosine and 2.37 g of N,N-diisopropylethylamine, and performing suction filtration after a reaction is completed; sequentially performing washing with dichloromethane, methanol and N,N-dimethylformamide, and then adding 100 mL of an N,N-dimethylformamide solution containing 20% of piperidine to remove an Fmoc group; after performing washing, adding 6.84 g of benzotriazole-N,N,N',N'-tetramethyluronium hexafluorophosphate, 0.23 g of 1-hydroxybenzotriazole, 10.55 g of Fmoc-S-triphenylmethyl-L-cysteine and 2.37 g of N,N-diisopropylethylamine, and performing suction filtration after a reaction is completed; sequentially performing washing with dichloromethane, methanol and N,N-dimethylformamide, and then adding 100 mL of an N,N-dimethylformamide solution containing 20% of piperidine to remove an Fmoc group; after performing washing, adding 6.84 g of benzotriazole-N,N,N',N'-tetramethyluronium hexafluorophosphate, 0.14 g of 1-hydroxybenzotriazole, 6.97 g of Fmoc-D-phenylalanine and 2.37 g of N,N-diisopropylethylamine, and performing suction filtration after a reaction is completed; sequentially performing washing with dichloromethane, methanol and N,N-dimethylformamide, adding 200 mL of N,N-dimethylformamide, then adding a mixture of 8.44 g of iodine, 10 mL of methanol and 10 mL of N,N-dimethylformamide to remove -TRT protection and realize cyclization, and adding 100 mL of an N,N-dimethylformamide solution containing 20% of piperidine to remove Fmoc protection; after performing washing, adding 60 mL of N,N-dimethylformamide, 3.42 g of 2-(7-azobenzotriazole)-N,N,N',N'-tetramethyluronium hexafluorophosphate, 1.16 g of N,N-diisopropylethylamine and 7.43 g of (S)-5-((4'-amino-3,3'-dimethyl-[1,1'-diphenyl]-4-yl)carbonyl)-1-(9H-fluoren-9-yl)-3,11-dioxo-2,1 4,17-trioxo-4,10-diazaicosane-20-acid, and performing suction filtration after a reaction is completed; sequentially performing washing with dichloromethane, methanol and N,N-dimethylformamide, and then adding 100 mL of an N,N-dimethylformamide solution containing 20% of piperidine to remove an Fmoc group; after performing washing, adding 60 mL of an N,N-dimethylformamide solution and 8.44 g of tri-tert-butyl 2,2',2"-(10-(2-((2,5-dioxopyrrolidin-1-yl)oxy)-2-oxoethyl)-1,4,7,10-tetraazacyclododecane-1,4,7-t riyl)triacetate, performing suction filtration after a reaction is completed, and then cutting a resin with 200 mL of a dichloromethane solution containing 20% of hexafluoroisopropanol; after performing spin-drying, adding acetonitrile, water and 4.5 mL of a 2 M HCl solution, adding 25 mL of N,N-dimethylformamide for dissolution, performing cooling, adding an aqueous solution of 0.75 g of sodium nitrite, adding 3.38 g of a 1-amino-8-naphthol-2,4-disulfonic acid monosodium salt and an aqueous solution of 11.34 g of sodium bicarbonate at low temperature, performing spin-drying after a reaction is completed, and performing purification by preparative separation to obtain an intermediate; and then adding the obtained intermediate to a 95% trifluoroacetic acid aqueous solution for deprotection, and performing purification by preparative liquid phase separation to obtain a target product.

4. A radionuclide labeled compound targeting SSTR2, wherein the compound is obtained by labeling the compound having the structure shown in the formula (II) according to claim 2 with a radionuclide.

5. The radionuclide labeled compound targeting SSTR2 according to claim 4, wherein the radioisotope is selected from isotopes emitting α rays, isotopes emitting β rays, isotopes emitting γ rays, isotopes emitting Auger electrons, or isotopes emitting X rays.

6. The radionuclide labeled compound targeting SSTR2 according to claim 4, wherein the radionuclide is selected from any one of ⁵¹Cr, ⁶⁴Cu, ⁶⁷Cu, ⁶⁷Ga, ^{6g}Ga, ⁸⁹Zr, ¹¹¹In, ^{99m}Tc, ¹⁸⁶Re, ¹⁸⁸Re, ¹³⁹La, ¹⁴⁰La, ¹⁷⁵Yb, ¹⁵³Sm, ¹⁶⁶Ho, ⁸⁶Y, ⁹⁰Y, ¹⁴⁹Pm, ¹⁶⁵Dy, ¹⁶⁹Er, ¹⁷⁷Lu, ⁴⁷Sc, ¹⁴²Pr, ¹⁵⁹Gd, ²¹²Bi, ²¹³Bi, ⁷²As, ⁷²Se, ⁹⁷Ru, ¹⁰⁹Pd, ¹⁰⁵Rh, ^{101m}Rh, ¹¹⁹Sb, ¹²⁸Ba, ¹²³I, ¹²⁴I, ¹³¹I, ¹⁹⁷Hg, ²¹¹At, ¹⁵¹Eu, ¹⁵³Eu, ¹⁶⁹Eu, ²⁰¹Tl, ²⁰³Pb, ²¹²Pb, ¹⁹⁸Au, ²²⁵Ac, ²²⁷Th, or ¹⁹⁹Ag.

7. The radionuclide labeled compound targeting SSTR2 according to claim 4, wherein the radionuclide is selected from ¹⁷⁷Lu, ²²⁵Ac, ⁶⁸Ga , ⁶⁴Cu, ⁸⁹Zr, ⁹⁰Y, ^{99m}Tc, ¹⁸⁸Re, or ¹¹¹In.

8. A method for preparing the radionuclide labeled compound targeting SSTR2 according to any one of claims 4-7, wherein the method comprises: enabling the compound targeting SSTR2 and capable of being labeled with a radionuclide according to claim 2 to undergo a reaction with a compound containing a radionuclide by a wet labeling method or a freeze-drying labeling method to prepare and obtain the radionuclide labeled compound targeting SSTR2.

9. A pharmaceutical composition, wherein the pharmaceutical composition comprises the compound targeting SSTR2 according to claim 1, the compound targeting SSTR2 and capable of being labeled with a radionuclide according to claim 2, the radionuclide labeled compound targeting SSTR2 according to any one of claims 4-7, or their pharmaceutically acceptable hydrates, solvates or salts.

10. A use of the compound targeting SSTR2 according to claim 1, the compound targeting SSTR2 and capable of being labeled with a radionuclide according to claim 2, the radionuclide labeled compound targeting SSTR2 according to claim 4, or their pharmaceutically acceptable hydrates, solvates or salts, or the pharmaceutical composition according to claim 9 in preparation of drugs for diagnosis or treatment of diseases **characterized by** over-expression of SSTR2 in animals or human individuals, wherein the diseases **characterized by** over-expression of SSTR2 are selected from: neuroendocrine tumors, gastroenteric tumors, lung cancer, hepatocellular carcinoma, head and neck cancer, ovarian cancer, myeloma, bladder cancer, clear cell renal cell carcinoma, carcinogenic osteomalacia, or sarcoma.

11. The use according to claim 10, wherein the neuroendocrine tumors are glioma.

12. The use according to claim 11, wherein the glioma is neuroglioma.

13. The use according to claim 12, wherein the neuroglioma is astrocytoma.

14. The use according to claim 10, wherein the gastroenteric tumors are selected from pancreatic cancer, small intestine cancer, colon cancer, rectal cancer, or cholangiocellular carcinoma.

15. The use according to claim 10, wherein the head and neck cancer is selected from nasopharyngeal carcinoma, esophageal cancer, hypopharyngeal carcinoma, or laryngeal carcinoma.
